# EUROPEAN PATENT SPECIFICATION

(11) **EP 4 340 911 B1**
(45) Date of publication and mention of the grant of the patent: **16.09.2026**
(21) Application number: 22728763.8
(22) Date of filing: 09.05.2022
(51) Int. Cl.: A61M 5/28, A61M 5/50, A61M 5/24, A61M 5/315, A61M 5/32

(54) **MEDICAMENT DELIVERY DEVICE**
MEDIKAMENTENVERABREICHUNGSVORRICHTUNG
DISPOSITIFS D'ADMINISTRATION DE MÉDICAMENTS

(30) Priority: 21.05.2021 EP 21175170
(43) Date of publication of application: 27.03.2024
(73) Proprietor: SHL Medical AG, 6302 Zug (CH)
(72) Inventor: CARLSSON, Daniel, 131 28 Nacka Strand (SE)
(86) International application number: PCT/EP2022/062461
(87) International publication number: WO 2022/243082

(56) References cited:
- US-A1- 2007 191 780
- US-A1- 2011 270 220
- US-A1- 2012 041 379
- US-A1- 2015 209 519
- US-A1- 2016 074 586
- US-B2- 9 656 027

## Description

### TECHNICAL FIELD

The present disclosure generally relates to medicament delivery devices.

### BACKGROUND

Some medicament dispensers such as certain types of syringes may have liquid containers that are mechanically flexible or soft. A blow-fill-seal (BFS) container is an example of a common type of flexible liquid container.

Today, BFS containers filled with a medicament are typically handled manually by the user during medicament delivery. This means that the user has to compress the liquid chamber manually to expel the medicament from the BGF container. Medicament delivery is usually done by medical professionals to ensure that the dose is administered correctly.

US2007/191780 describes a device for intradermal delivery, the device comprising a housing that contains a reservoir chamber in which a flexible reservoir is placed.

### SUMMARY

An object of the present disclosure is to provide a medicament delivery device which solves, or at least mitigates problems of the prior art.

There is hence provided a medicament delivery device comprising: a housing, an actuator structure arranged in the housing and configured to be moved linearly from an initial position relative to the housing to a final position, the actuator structure having a medicament container holder configured to receive a mechanically flexible medicament container, the actuator structure comprising a movable button arranged in a default position protruding from the housing when the actuator structure is in the initial position, wherein the button is configured to be moved from the default position into the medicament container holder to a squeeze position to squeeze the medicament container, wherein the actuator structure has a first engagement structure and the housing has a second engagement structure configured to engage with the first engagement structure when the button is in the default position, to prevent the actuator structure from movement from the initial position towards the final position, wherein the first engagement structure is configured to disengage from engagement with the second engagement structure when the button is moved towards the squeeze position, enabling the actuator structure to be moved from the initial position to the final position.

Medicament delivery from a mechanically flexible medicament container may thus be provided by means of the medicament delivery device. Medicament administration may thus be facilitated, especially for non-professional users.

Moreover, movement of the actuator structure from the initial position to the final position causes movement of the medicament container holder relative to the housing. Thus, the medicament container arranged in the medicament container holder is also moved with the actuator structure when the button has been moved to the squeeze position and the actuator structure is moved from the initial position to the final position.

In the present disclosure, when the term "distal direction" is used, this refers to the direction pointing away from the dose delivery site during use of the medicament delivery device. When the term "distal part/end" is used, this refers to the part/end of the delivery device, or the parts/ends of the members thereof, which under use of the medicament delivery device is/are located furthest away from the dose delivery site. Correspondingly, when the term "proximal direction" is used, this refers to the direction pointing towards the dose delivery site during use of the medicament delivery device. When the term "proximal part/end" is used, this refers to the part/end of the delivery device, or the parts/ends of the members thereof, which under use of the medicament delivery device is/are located closest to the dose delivery site.

Further, the term "longitudinal", "longitudinally", "axially" or "axial" refer to a direction extending from the proximal end to the distal end, typically along the device or components thereof in the direction of the longest extension of the device and/or component.

Similarly, the terms "transverse", "transversal" and "transversally" refer to a direction generally perpendicular to the longitudinal direction.

Further, the terms "circumference", "circumferential", or "circumferentially" refer to a circumference or a circumferential direction relative to an axis, typically a central axis extending in the direction of the longest extension of the device and/or component. Similarly, "radial" or "radially" refer to a direction extending radially relative to the axis, and "rotation", "rotational" and "rotationally" refer to rotation relative to the axis.

According to one embodiment the housing has a radial opening through which the button extends in the default position (and optionally also in the squeeze position), wherein the radial opening has an axial extension which is larger than the corresponding dimension of the button to allow the actuator structure to move linearly from the initial position to the final position by linear movement of the button when the button is in the squeeze position.

The button may be multi-functional. The button may in particular be used both for squeezing the medicament container to discharge medicament and subsequently for moving the actuator structure from the initial position to the final position. Medicament delivery and displacement of the actuator structure may thus be performed by a simple sequence of user movements without having to change grip on the button, for example by the use of the thumb.

According to one embodiment the actuator structure has a third engagement structure and the housing has a fourth engagement structure, wherein the third engagement structure is configured to axially align and engage with the fourth engagement structure when the actuator structure is in the final position, interlocking the actuator structure with the housing.

The actuator structure is thus locked in the final position.

According to one embodiment the third engagement structure and the fourth engagement structure are first snap fit connectors.

According to one embodiment the first snap fit connectors are cantilever snap fit connectors.

According to one embodiment the housing has a proximal end provided with a delivery member opening, and a distal end opposite to the proximal end, wherein the actuator structure is moved towards the distal end when the actuator structure is moved from the initial position towards the second position.

The actuator structure is thus locked in the final position, in which the actuator structure has moved further into the housing towards the distal end.

The medicament container may be connected to a delivery member which is retracted into the housing by movement of the actuator structure to the final position, locking the delivery member in a position inside the housing. The delivery member, which may be for example a needle, may thereby be fully retracted into and axially locked inside the housing. This can allow anyone handing the medicament delivery device after medicament delivery to be protected from the used delivery member. It can moreover signal to users that the medicament delivery device has been used.

According to one embodiment movement of the actuator structure causes retraction of a delivery member into the housing through the delivery member opening.

According to one embodiment the button is a lever. The movement from the default position to the squeeze position may be a rotational motion. The button may thus be able to first move by rotation and subsequently by translation when the actuator structure is moved from the initial position to the final position rectilinearly.

According to one embodiment the first engagement structure and the second engagement structure are second snap fit connectors.

According to one embodiment the second snap fit connectors are torsional snap fit connectors.

According to one embodiment the second engagement structure has a see-saw functionality which in a first see-saw position engages with the second engagement structure.

According to one embodiment the button has a protrusion configured to cooperate with second engagement structure to tip the second engagement structure to a second see-saw position when the button is moved from the default position to the squeeze position.

According to one embodiment the housing has a radial opening through which the button extends in the default position (and optionally also in the squeeze position), wherein the first engagement structure forms part of a distal end of the button and a distal edge of the radial opening defines the second engagement structure.

According to one embodiment the button is configured to cooperate with the second engagement structure when the button is moved towards the squeeze position, disengaging the second engagement structure from engagement with the first engagement structure.

Generally, all terms used in the claims are to be interpreted according to their ordinary meaning in the technical field, unless explicitly defined otherwise herein. All references to "a/an/the member, apparatus, component, means, etc." are to be interpreted openly as referring to at least one instance of the member, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 shows a perspective view of an example of a medicament delivery device before use;
Fig. 2 is an exploded view of the medicament delivery device in Fig. 1;
Fig. 3 is a longitudinal section of the medicament delivery device shown in Fig. 1;
Fig. 4 is a longitudinal section of the medicament delivery device when the button has moved from the default position to the squeeze position;
Fig. 5 is a perspective view showing the medicament delivery device in the same state as in Fig. 4;
Fig. 6 is a longitudinal section of the medicament delivery device when the actuator structure is in the final position; and
Fig. 7 shows a perspective view of the medicament delivery device in the same state as in Fig. 6.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like members throughout the description.

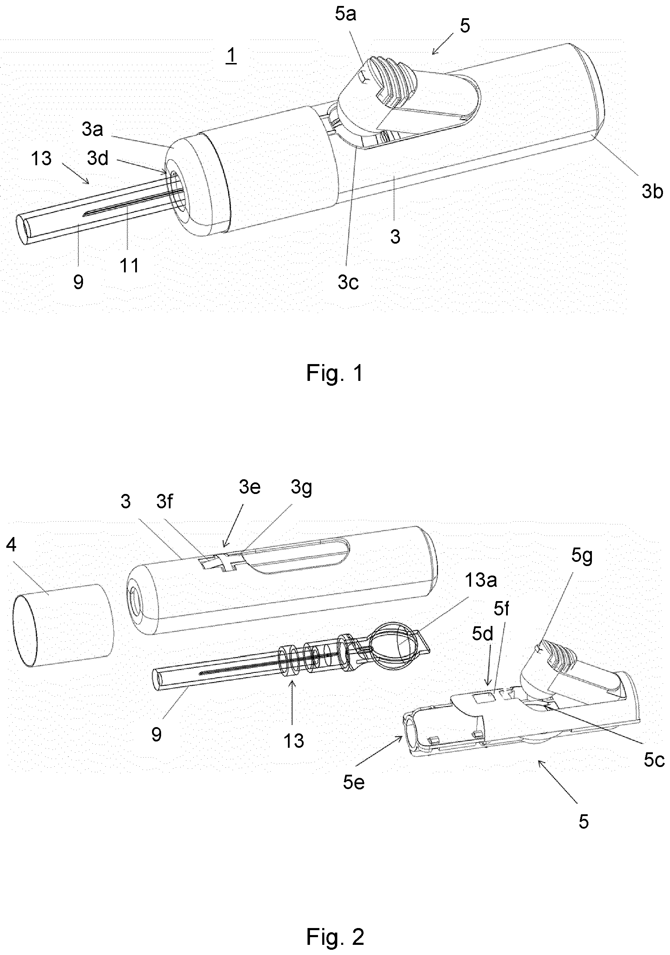

Fig. 1 shows an example of a medicament delivery device 1 configured for use with a syringe having a mechanically flexible medicament container.

The syringe may for example be a blow-fill-seal type of syringe. The medicament delivery device 1 could alternatively be configured to be used with other types of dispensing devices having a mechanically flexible medicament container.

The medicament delivery device 1 may be disposable.

The medicament delivery device 1 comprises a housing 3. The housing 3 may be elongated. The housing 3 has a proximal end 3a and a distal end 3b. The housing 3 has a longitudinal axis which extends between the proximal end 3a and the distal end 3b.

The medicament delivery device 1 comprises an actuator structure 5 arranged in the housing 3. The actuator structure comprises a button 5a extending from the housing 3. The housing 3 has a radial opening, or window, 3c through which the button 3a extends from the housing 3.

The button 5a is configured to be moved from a default position, shown in Fig. 1, to a squeeze position in which the button 5a is moved further into the housing 3 through the radial opening 3c. The button 5a is in the present example a lever. The button 5a could alternatively be configured to move linearly in the radial direction.

The actuator structure 5 is configured to be moved relative to the housing 3 from an initial position, shown in Fig. 1, to a final position. The axial length of the actuator structure 5 is shorter than the axial distance between the proximal end 3a and the distal end 3b of the housing 3. When the actuator structure 5 is in the initial position, there is a spacing between the distal end of the actuator structure 5 and the distal end 3b of the housing 3. The actuator structure 5 is thereby able to slide towards the distal end 3b when moved towards the final position.

The actuator structure 5 is locked in the initial position when the button 5a is in the default position. The actuator structure 5 is disengaged from engagement with the housing 3 when the button 5a is moved to the squeeze position.

The medicament delivery device 1 depicted in Fig. 1 is provided with a syringe 13. The syringe 13 is arranged in the housing 3. The syringe 13 has a mechanically flexible medicament container.

The actuator structure 5 has a medicament container holder configured to receive the mechanically flexible medicament container. The medicament container holder is arranged aligned with the radial opening 3c in the housing 3 so that when the button 5a is moved from the default position towards the squeeze position, the button 5a moves into the medicament container holder and squeezes the medicament container.

The proximal end 3a is provided with a delivery member opening 3d. The delivery member opening 3d is a through-opening that extends into the housing 3.

The exemplified syringe 13 comprises a removable needle shield 9, e.g. a rigid needle shield, RNS, extending through the delivery member opening 3d before the medicament delivery device 1 has been used. The syringe 13 has a delivery member 11, in the present example a needle, which is covered by the needle shield 9. The delivery member 11 extends through the deliver member opening 3d before the medicament delivery device 1 has been used. Prior to use, the needle shield 9 is removed from the delivery member 11, exposing the delivery member 11.

Fig. 2 shows an exploded view of the medicament delivery device 1. The medicament container holder 5c is here depicted. The actuator structure 5 comprises a proximal opening 5e which is connected to the medicament container holder 5c and which is aligned with the delivery member opening 3d of the housing 3. The proximal opening 5e is configured to receive a portion of the needle shield 9.

The actuator structure 5 comprises a first engagement structure 5d. The housing 3 comprises a second engagement structure 3e. The first engagement structure 5d is configured to engage with the second engagement structure 3e when the button 5a is in the default position. The actuator structure 5 is thereby locked in the initial position relative to the housing 3.

The first engagement structure 5d and the second engagement structure 3e may be snap fit connectors, herein referred to as second snap fit connectors. The second snap fit connectors may for example be torsional snap fit connectors.

In the example shown in Fig. 2, the first engagement structure 5d comprises a fulcrum 5f and openings or cut-outs provided on either side of the fulcrum 5f in the axial direction of the actuator structure 5.

The exemplified second engagement structure 3e has a see-saw structure. The second engagement structure has a see-saw functionality. The second engagement structure 3e has seesaw arms 3f and 3g.

The fulcrum 5f is configured to interact with the seesaw arms 3f and 3g. In a first see-saw position of the second engagement structure 3e, the proximal seesaw arm 3f of the two seesaw arms 3f and 3g is tilted so that it engages with the second engagement structure 5d, preventing the actuator structure 5 to move towards the final position. The proximal seesaw arm 3f is tilted radially inwards of the fulcrum 5f, which thereby forms a radial stop surface for the second engagement structure 3e and prevents axial movement of the actuator structure 5 relative to the housing 3.

The button 5a has a protrusion 5g. The protrusion 5g extends in the proximal direction. The protrusion 5g is configured to cooperate with the distal seesaw arm 3g when the button 5a is moved towards the squeeze position, causing the distal seesaw arm 3g to tilt radially inwards and the proximal seesaw arm 3f to tilt radially outwards. The second engagement structure 3e is thus tipped to a second see-saw position. The first engagement structure 5d and the second engagement structure 3e are thereby disengaged. The actuator structure 5 is thus disengaged from engagement with the housing 3.

There are other alternative ways to cause disengagement of the actuator structure from the housing by pressing the button towards the squeeze position than employing the above-described see-saw structure. According to one example, the medicament delivery device does not have any see-saw structure and fulcrum. Instead, the first engagement structure forms part of a distal end of the button and a distal edge of the radial opening defines the second engagement structure. Hereto, the actuator structure disengages from the housing by folding, or pushing, the button into the radial opening of the housing such that the distal end moves radially below the level of the distal edge of the radial opening. The button would in itself thus block the actuator structure from moving from the initial position to the final position as long as the button is in the default position.

The medicament delivery device 1 may comprise a sleeve 4 configured to be arranged on the housing 3 over the second engagement structure 3e as a cover.

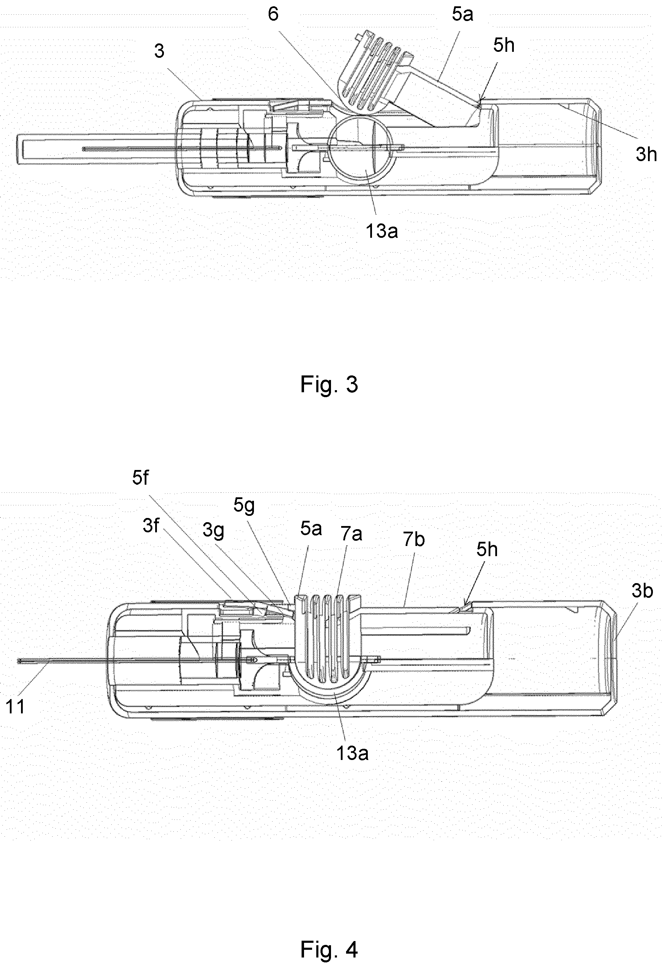

The operation of the medicament delivery device 1 will now be explained with reference to Figs 3-7.

Fig. 3 shows a longitudinal section of the medicament delivery device 1 when the button 5a is in the default position and the actuator structure 5 is in the initial position. This is a state before use of the medicament delivery device 1. The first engagement structure 5d and the second engagement structure 3e are engaged and the actuator structure 5 is thus locked in the initial position.

The medicament container 13a is arranged in the medicament container holder 5c, axially aligned with the radial opening 3c of the housing 3. The medicament container holder 5c and the medicament container 13a are aligned with the button 5a.

As shown in Fig. 3, the button 5a may have an inner surface 6 corresponding to the shape of the interior of the medicament container holder 5c. The inner surface 6 is in the present example rounded. The inner surface 6 may be configured to mate with the interior of the medicament container holder 5c when the button 5a is in the squeeze position. It may thus be ensured that essentially the entire content of the medicament container 13a can be emptied. The medicament container 13a may according to one variation have a spherical or half-spherical bottom shape corresponding to the curvature of the inner surface 6.

The actuator structure 5 comprises a third engagement structure 5h and the housing 3 comprises a fourth engagement structure 3h configured to engage with each other when the actuator structure 5 is in the final position. The actuator structure 5 and the housing 3 are thereby interlocked with each other when the actuator structure 5 is in the final position. Provision of a third engagement structure and a fourth engagement structure can be beneficial to lock the device after use, but is optional - an alternative locking structure could be provided elsewhere on the device, or even no lock at all.

The third engagement structure 5h and the fourth engagement structure 3h may be snap fit connectors, herein referred to as first snap fit connectors. The first snap fit connectors may for example be cantilever snap fit connectors.

In the example shown in Fig. 3, the third engagement structure 5h is a recess or opening and the fourth engagement structure 3h is a protrusion extending radially inwards from the inner surface of the housing. The protrusion may be wedge-shaped with its inclined surface facing the proximal end 3a of the housing 3.

Alternatively, the recess or opening could be provided in the housing and a radially outwards extending protrusion could be provided on the actuator structure, for example on a radially flexible tongue. According to another alternative, the second engagement structure could also be the fourth engagement structure. In this case, the actuator structure would have to be designed such that the distal seesaw arm would move radially inwards when the actuator structure has reached its final position and the actuator structure would have a proximal radial surface bearing against the distal seesaw arm, which would thus block the actuator structure from moving proximally from the final position.

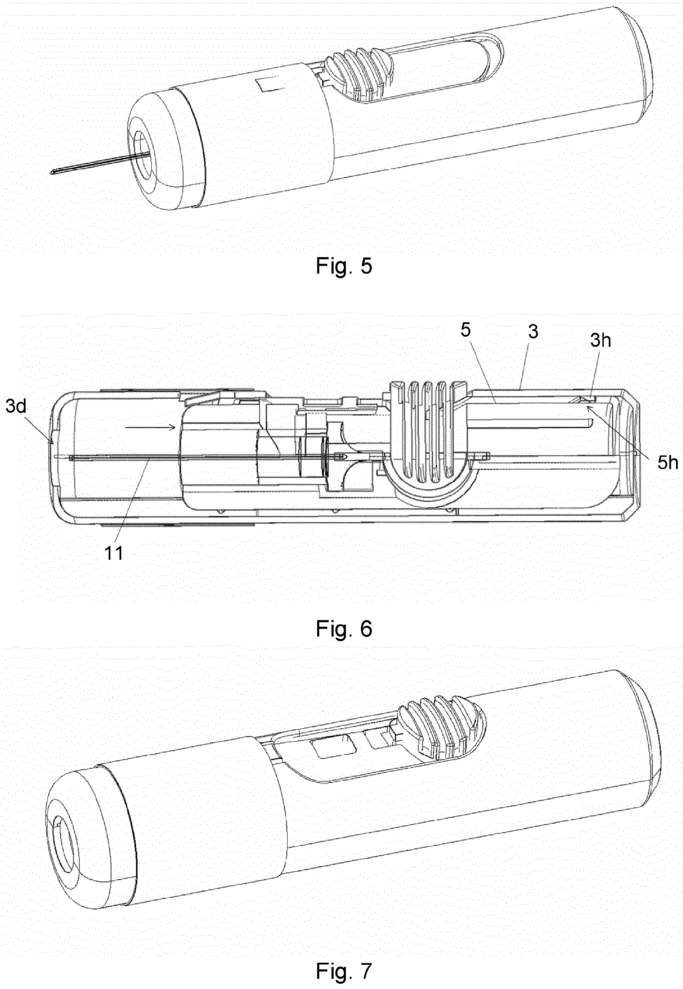

Figs 4 and 5 show the medicament delivery device 1 after the needle shield 9 has been removed from the delivery member 11 and the button 5a has been moved further into the housing 3 to the squeeze position. The actuator structure 5 is still in the initial position.

The button 5a has a proximal outer contact surface 7a which is arranged close to the proximal edge of the radial opening 3c of the housing 3 as long as the actuator structure 5 is in the initial position. The outer contact surface 7a protrudes from the radial opening 3c when the button 5a is in the default position and in the squeeze position. The outer contact surface 7a is smaller in axial dimension than the length of the radial opening 3c. The length of the radial opening 3c is the distance between the proximal edge and the distal edge. The button 5a has a distal outer surface 7b arranged distally relative to the outer contact surface 7a and radially below the inner surface of the housing 3 when the button 5a is in the squeeze position to enable sliding of the actuator structure 5 towards the distal end 3b of the housing 3.

In Fig. 4, the button 5a has compressed the mechanically flexible medicament container 13a. The medicament contained in the medicament container 13a has thus been discharged through the delivery member 11.

The protrusion 5g presses the distal seesaw arm 3g radially inwards, causing the proximal seesaw arm 3f to tilt radially outwards and to disengage from contact with the fulcrum 5f. The first engagement structure 5d is thus disengaged from the second engagement structure 3e and the actuator structure 5 is thus disengaged from the housing 3.

The third engagement structure 5h, here exemplified by a recess, is here more clearly shown.

Figs 6 and 7 show the medicament delivery device 1 after medicament delivery has been completed. The actuator structure 5 has been moved from the initial position to the final position. The delivery member 11 has thus been retracted fully into the housing 3 through the delivery member opening 3d. The third engagement structure 5h has moved into axial alignment with the fourth engagement structure 3h. The fourth engagement structure 3h thus engages with the third engagement structure 5h. The actuator structure 5 is thereby axially interlocked with the housing 3.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A medicament delivery device (1) comprising:
a housing (3),
an actuator structure (5) arranged in the housing (3) and configured to be moved linearly from an initial position relative to the housing (3) to a final position,
the actuator structure (5) having a medicament container holder (5c) configured to receive a mechanically flexible medicament container (13a),
the actuator structure (5) comprising a movable button (5a) arranged in a default position protruding from the housing (3) when the actuator structure (5) is in the initial position, wherein the button (5a) is configured to be moved from the default position into the medicament container holder (5c) to a squeeze position to squeeze the medicament container (13a),
wherein the actuator structure (5) has a first engagement structure (5d) and the housing (3) has a second engagement structure (3e) configured to engage with the first engagement structure (5d) when the button (5a) is in the default position, to prevent the actuator structure (5) from movement from the initial position towards the final position,
wherein the first engagement structure (5d) is configured to disengage from engagement with the second engagement structure (3e) when the button (5a) is moved towards the squeeze position, enabling the actuator structure (5) to be moved from the initial position to the final position,
wherein the housing (3) has a radial opening (3c) through which the button (5a) extends in the default position, wherein the first engagement structure forms part of a distal end of the button (5a) and a distal edge of the radial opening (3c) defines the second engagement structure (3e).

2. The medicament delivery device (1) as claimed in claim 1, wherein the radial opening (3c) has an axial extension which is larger than the corresponding dimension of the button (5a) to allow the actuator structure (5) to move linearly from the initial position to the final position by linear movement of the button (5a) when the button (5a) is in the squeeze position.

3. The medicament delivery device (1) as claimed in claim 1 or 2, wherein the actuator structure (5) has a third engagement structure (5h) and the housing (3) has a fourth engagement structure (3h), wherein the third engagement structure (5g) is configured to axially align and engage with the fourth engagement structure (3h) when the actuator structure (5) is in the final position, interlocking the actuator structure (5) with the housing (3).

4. The medicament delivery device (1) as claimed in claim 3, wherein the third engagement structure (5h) and the fourth engagement structure (3h) are first snap fit connectors.

5. The medicament delivery device (1) as claimed in claim 4, wherein the first snap fit connectors are cantilever snap fit connectors.

6. The medicament delivery device (1) as claimed in any of the preceding claims, wherein the housing (3) has a proximal end (3a) provided with a delivery member opening (3d), and a distal end (3b) opposite to the proximal end (3a), wherein the actuator structure (5) is moved towards the distal end (3b) when the actuator structure (5) is moved from the initial position towards the second position.

7. The medicament delivery device (1) as claimed in claim 6, wherein movement of the actuator structure (5) causes retraction of a delivery member (11) into the housing (3) through the delivery member opening (3d).

8. The medicament delivery device (1) as claimed in any of the preceding claims, wherein the button (5a) is a lever.

9. The medicament delivery device (1) as claimed in any of the preceding claims, wherein the first engagement structure (5d) and the second engagement structure (3e) are second snap fit connectors.

10. The medicament delivery device (1) as claimed in claim 9, wherein the second snap fit connectors are torsional snap fit connectors.

11. The medicament delivery device (1) as claimed in claim 10, wherein the second engagement structure (3e) has a see-saw functionality which in a first see-saw position engages with the second engagement structure (5d).

12. The medicament delivery device (1) as claimed in claim 11, wherein the button (5a) has a protrusion (5g) configured to cooperate with second engagement structure (3e) to tip the second engagement structure (3e) to a second see-saw position when the button (5a) is moved from the default position to the squeeze position.

13. The medicament delivery device (1) as claimed in any of claims 1-10, wherein the button (5a) is configured to cooperate with the second engagement structure (3e) when the button (5a) is moved towards the squeeze position, disengaging the second engagement structure (3e) from engagement with the first engagement structure (5d).

## Patentansprüche

1. Medikamentenabgabevorrichtung (1), umfassend:
ein Gehäuse (3),
eine Betätigungselementstruktur (5), die in dem Gehäuse (3) angeordnet und konfiguriert ist, um linear von einer Ausgangsposition relativ zu dem Gehäuse (3) in eine Endposition bewegt zu werden,
wobei die Betätigungselementstruktur (5) einen Medikamentenbehälterhalter (5c) aufweist, der konfiguriert ist, um einen mechanisch flexiblen Medikamentenbehälter (13a) aufzunehmen,
die Betätigungselementstruktur (5) umfassend einen bewegbaren Knopf (5a), der in einer Standardposition angeordnet ist, die aus dem Gehäuse (3) herausragt, wenn sich die Betätigungselementstruktur (5) in der Ausgangsposition befindet, wobei der Knopf (5a) konfiguriert ist, um aus der Standardposition in den Medikamentenbehälterhalter (5c) in eine Zusammendrückposition bewegt zu werden, um den Medikamentenbehälter (13a) zusammenzudrücken,
wobei die Betätigungselementstruktur (5) eine erste Eingriffsstruktur (5d) aufweist und das Gehäuse (3) eine zweite Eingriffsstruktur (3e) aufweist, die konfiguriert ist, um mit der ersten Eingriffsstruktur (5d) in Eingriff zu stehen, wenn sich der Knopf (5a) in der Standardposition befindet, um die Betätigungselementstruktur (5) an einer Bewegung von der Ausgangsposition in Richtung der Endposition zu hindern,
wobei die erste Eingriffsstruktur (5d) konfiguriert ist, um sich aus dem Eingriff mit der zweiten Eingriffsstruktur (3e) zu lösen, wenn der Knopf (5a) in Richtung der Zusammendrückposition bewegt wird, wodurch ermöglicht wird, dass die Betätigungselementstruktur (5) von der Ausgangsposition in die Endposition bewegt wird,
wobei das Gehäuse (3) eine radiale Öffnung (3c) aufweist, durch die sich der Knopf (5a) in der Standardposition erstreckt, wobei die erste Eingriffsstruktur einen Teil eines distalen Endes des Knopfes (5a) ausbildet und eine distale Kante der radialen Öffnung (3c) die zweite Eingriffsstruktur (3e) definiert.

2. Medikamentenabgabevorrichtung (1) nach Anspruch 1, wobei die radiale Öffnung (3c) eine axiale Erstreckung aufweist, die größer als die entsprechende Abmessung des Knopfes (5a) ist, um zu ermöglichen, dass sich die Betätigungselementstruktur (5) durch eine lineare Bewegung des Knopfes (5a) linear von der Ausgangsposition in die Endposition bewegt, wenn sich der Knopf (5a) in der Zusammendrückposition befindet.

3. Medikamentenabgabevorrichtung (1) nach Anspruch 1 oder 2, wobei die Betätigungselementstruktur (5) eine dritte Eingriffsstruktur (5h) aufweist und das Gehäuse (3) eine vierte Eingriffsstruktur (3h) aufweist, wobei die dritte Eingriffsstruktur (5g) konfiguriert ist, um sich axial mit der vierten Eingriffsstruktur (3h) auszurichten und in diese einzugreifen, wenn sich die Betätigungselementstruktur (5) in der Endposition befindet, wodurch die Betätigungselementstruktur (5) mit dem Gehäuse (3) verriegelt wird.

4. Medikamentenabgabevorrichtung (1) nach Anspruch 3, wobei die dritte Eingriffsstruktur (5h) und die vierte Eingriffsstruktur (3h) erste Schnappverbinder sind.

5. Medikamentenabgabevorrichtung (1) nach Anspruch 4, wobei die ersten Schnappverbinder Kragarmschnappverbinder sind.

6. Medikamentenabgabevorrichtung (1) nach einem der vorstehenden Ansprüche, wobei das Gehäuse (3) ein proximales Ende (3a), das mit einer Abgabeelementöffnung (3d) versehen ist, und ein distales Ende (3b) gegenüber dem proximalen Ende (3a) aufweist, wobei die Betätigungselementstruktur (5) in Richtung des distalen Endes (3b) bewegt wird, wenn die Betätigungselementstruktur (5) von der Ausgangsposition in Richtung der zweiten Position bewegt wird.

7. Medikamentenabgabevorrichtung (1) nach Anspruch 6, wobei die Bewegung der Betätigungselementstruktur (5) ein Zurückziehen eines Abgabeelements (11) in das Gehäuse (3) durch die Abgabeelementöffnung (3d) bewirkt.

8. Medikamentenabgabevorrichtung (1) nach einem der vorstehenden Ansprüche, wobei der Knopf (5a) ein Hebel ist.

9. Medikamentenabgabevorrichtung (1) nach einem der vorstehenden Ansprüche, wobei die erste Eingriffsstruktur (5d) und die zweite Eingriffsstruktur (3e) zweite Schnappverbinder sind.

10. Medikamentenabgabevorrichtung (1) nach Anspruch 9, wobei die zweiten Schnappverbinder Torsionsschnappverbinder sind.

11. Medikamentenabgabevorrichtung (1) nach Anspruch 10, wobei die zweite Eingriffsstruktur (3e) eine Wippenfunktionalität aufweist, die in einer ersten Wippenposition in die zweite Eingriffsstruktur (5d) eingreift.

12. Medikamentenabgabevorrichtung (1) nach Anspruch 11, wobei der Knopf (5a) einen Vorsprung (5g) aufweist, der konfiguriert ist, um mit der zweiten Eingriffsstruktur (3e) zusammenzuwirken, um die zweite Eingriffsstruktur (3e) in eine zweite Wippenposition zu kippen, wenn der Knopf (5a) von der Ausgangsposition in die Zusammendrückposition bewegt wird.

13. Medikamentenabgabevorrichtung (1) nach einem der Ansprüche 1 bis 10, wobei der Knopf (5a) konfiguriert ist, um mit der zweiten Eingriffsstruktur (3e) zusammenzuwirken, wenn der Knopf (5a) in Richtung der Zusammendrückposition bewegt wird, wodurch die zweite Eingriffsstruktur (3e) aus dem Eingriff mit der ersten Eingriffsstruktur (5d) gelöst wird.

## Revendications

1. Dispositif d'administration de médicament (1) comprenant :
un logement (3),
une structure d'actionneur (5) agencée dans le logement (3) et conçue pour être déplacée linéairement d'une position initiale par rapport au logement (3) à une position finale,
la structure d'actionneur (5) ayant un support de récipient de médicament (5c) conçu pour recevoir un récipient de médicament (13a) mécaniquement flexible,
la structure d'actionneur (5) comprenant un bouton mobile (5a) agencé dans une position par défaut faisant saillie du logement (3) lorsque la structure d'actionneur (5) est dans la position initiale, dans lequel le bouton (5a) est conçu pour être déplacé de la position par défaut dans le support de récipient de médicament (5c) à une position de pression pour presser le récipient de médicament (13a),
dans lequel la structure d'actionneur (5) a une première structure de mise en prise (5d) et le logement (3) a une deuxième structure de mise en prise (3e) conçue pour venir en prise avec la première structure de mise en prise (5d) lorsque le bouton (5a) est dans la position par défaut, pour empêcher la structure d'actionneur (5) d'entrer en mouvement de la position initiale vers la position finale,
dans lequel la première structure de mise en prise (5d) est conçue pour se mettre hors de prise de la mise en prise avec la deuxième structure de mise en prise (3e) lorsque le bouton (5a) est déplacé vers la position de pression, permettant à la structure d'actionneur (5) d'être déplacée de la position initiale à la position finale,
dans lequel le logement (3) a une ouverture radiale (3c) à travers laquelle le bouton (5a) s'étend dans la position par défaut, dans lequel la première structure de mise en prise forme une partie d'une extrémité distale du bouton (5a) et un bord distal de l'ouverture radiale (3c) définit la deuxième structure de mise en prise (3e).

2. Dispositif d'administration de médicament (1) selon la revendication 1, dans lequel l'ouverture radiale (3c) a une extension axiale qui est plus grande que la dimension correspondante du bouton (5a) pour permettre à la structure d'actionneur (5) d'entrer en mouvement linéairement de la position initiale à la position finale par un mouvement linéaire du bouton (5a) lorsque le bouton (5a) est dans la position de pression.

3. Dispositif d'administration de médicament (1) selon la revendication 1 ou 2, dans lequel la structure d'actionneur (5) a une troisième structure de mise en prise (5h) et le logement (3) a une quatrième structure de mise en prise (3h), dans lequel la troisième structure de mise en prise (5g) est conçue pour s'aligner axialement et venir en prise avec la quatrième structure de mise en prise (3h) lorsque la structure d'actionneur (5) est dans la position finale, verrouillant mutuellement la structure d'actionneur (5) avec le logement (3).

4. Dispositif d'administration de médicament (1) selon la revendication 3, dans lequel la troisième structure de mise en prise (5h) et la quatrième structure de mise en prise (3h) sont des premiers raccords à encliquetage.

5. Dispositif d'administration de médicament (1) selon la revendication 4, dans lequel les premiers raccords à encliquetage sont des raccords à encliquetage en porte-à-faux.

6. Dispositif d'administration de médicament (1) selon l'une quelconque des revendications précédentes, dans lequel le logement (3) a une extrémité proximale (3a) pourvue d'une ouverture d'élément d'administration (3d), et une extrémité distale (3b) opposée à l'extrémité proximale (3a), dans lequel la structure d'actionneur (5) est déplacée vers l'extrémité distale (3b) lorsque la structure d'actionneur (5) est déplacée de la position initiale vers la seconde position.

7. Dispositif d'administration de médicament (1) selon la revendication 6, dans lequel un mouvement de la structure d'actionnement (5) entraîne une rétraction d'un élément d'administration (11) dans le logement (3) à travers l'ouverture d'élément d'administration (3d).

8. Dispositif d'administration de médicament (1) selon l'une quelconque des revendications précédentes, dans lequel le bouton (5a) est un levier.

9. Dispositif d'administration de médicament (1) selon l'une quelconque des revendications précédentes, dans lequel la première structure de mise en prise (5d) et la deuxième structure de mise en prise (3e) sont des seconds raccords à encliquetage.

10. Dispositif d'administration de médicament (1) selon la revendication 9, dans lequel les seconds raccords à encliquetage sont des raccords à encliquetage de torsion.

11. Dispositif d'administration de médicament (1) selon la revendication 10, dans lequel la deuxième structure de mise en prise (3e) a une fonctionnalité de bascule qui, dans une première position de bascule, vient en prise avec la deuxième structure de mise en prise (5d).

12. Dispositif d'administration de médicament (1) selon la revendication 11, dans lequel le bouton (5a) a une saillie (5g) conçue pour coopérer avec la deuxième structure de mise en prise (3e) pour incliner la deuxième structure de mise en prise (3e) dans une seconde position de bascule lorsque le bouton (5a) est déplacé de la position par défaut à la position de pression.

13. Dispositif d'administration de médicament (1) selon l'une quelconque des revendications 1 à 10, dans lequel le bouton (5a) est conçu pour coopérer avec la deuxième structure de mise en prise (3e) lorsque le bouton (5a) est déplacé vers la position de pression, mettant hors de prise la deuxième structure de mise en prise (3e) de la mise en prise avec la première structure de mise en prise (5d).
